# FASCICULE DE BREVET EUROPEEN

(11) **EP 1 485 045 B1**
(45) Date de publication et mention de la délivrance du brevet: **01.07.2015**
(21) Numéro de dépôt: 03725283.0
(22) Date de dépôt: 13.03.2003
(51) Int. Cl.: A61F 2/44, A61F 2/30, A61B 17/70, A61B 17/86, A61F 2/28

(54) **IMPLANT INTERVERTEBRAL DYNAMIQUE**
DYNAMISCHES ZWISCHENWIRBELIMPLANTAT
DYNAMIC INTERVERTEBRAL IMPLANT

(30) Priorité: 15.03.2002 FR 0203252
(43) Date de publication de la demande: 15.12.2004
(73) Titulaire: Paradigm Spine, LLC, Wilmington, DE 19808 (US)
(72) Inventeur: MATGE, Guy, 8229 MAMER (LU); MARTIN, Jean-Jacques, 01000 BOURG EN BRESSE (FR)
(74) Mandataire: Schulz, Oliver Frank Michael
(86) Numéro de dépôt international: PCT/FR2003/000799
(87) Numéro de publication internationale: WO 2003/077806

(56) Documents cités:
- WO-A-00/04851
- WO-A-01/62190
- FR-A- 2 812 806
- US-A- 5 306 307
- US-A- 5 713 899
- US-A- 5 749 916
- US-B1- 6 235 059

## Description

La présente invention concerne un implant intervertébral, notamment destiné au traitement de vertèbres cervicales par voie d'abord antérieur.

Il est connu d'utiliser des implants intervertébraux pour restaurer l'espace intervertébral anatomique entre deux vertèbres. Toutefois, les implants existants ne donnent pas parfaitement satisfaction, particulièrement en ce qui concerne le traitement de vertèbres cervicales par voie d'abord antérieur, soit qu'ils ne restaurent pas parfaitement l'espace intervertébral, soit qu'ils forment des obstacles aux mouvements des vertèbres, soit qu'ils induisent des risques d'insertion dans les plateaux vertébraux, soit qu'ils sont difficiles à implanter, soit que leur pérennité ou celle de leur ancrage est sujette à caution.

Le document US 5 749 916 décrit une cage de fusion fendue latéralement, pour permettre l'exercice de contraintes sur un greffon contenu dans la cale et/ou la restauration de la mobilité anatomique entre deux vertèbres.

Cet implant n'est pas destiné au traitement de vertèbres cervicales par voie d'abord antérieur, et l'implant selon l'invention ne comprend pas une telle fente latérale.

Il est par ailleurs connu, par le document WO 01/62190, un implant intervertébral comprenant un corps en forme de U vu de profil, c'est-à-dire présentant deux branches latérales d'appui contre les plateaux verticaux et un "mur" postérieur. Ce corps est déformable élastiquement pour son insertion entre les vertèbres à traiter et pour permettre de restaurer la mobilité des vertèbres, et forme des pattes saillantes pour sa fixation aux vertèbres.

Cet implant est estimé comme ne donnant pas satisfaction du point de vue de la restauration d'un espace intervertébral avec mobilité des vertèbres. En effet, la fixation par vis de cet implant est considérée comme non adaptée à une telle restauration, compte tenu des risques qu'elle induit d'une fusion vertébrale par croissance des cellules osseuses, de laquelle il peut résulter une immobilisation des vertèbres. De plus, la résistance de cet implant aux sollicitations répétées transmises par ces vertèbres est considérée comme sujette à caution.

La présente invention vise à remédier à ces inconvénients.

Son objectif principal est donc de fournir un implant intervertébral à même de restaurer un espace anatomique adéquat entre deux vertèbres tout en conservant, de manière certaine dans le temps, la mobilité relative des deux vertèbres traitées.

Un autre objectif de l'invention est de fournir un implant intervertébral ayant une parfaite résistance aux sollicitations répétées transmises par ces vertèbres.

L'implant concerné est défini par la revendication 1 et comprend, comme cela est connu, deux parois latérales d'appui contre les plateaux vertébraux et une paroi intermédiaire de raccordement de ces parois latérales d'appui, cet implant étant déformable élastiquement pour son insertion entre les vertèbres à traiter et pour permettre de restaurer une mobilité amortie de ces vertèbres, et comprenant des moyens pour son assemblage à ces vertèbres.

Selon la revendication 1,
- lesdites parois latérales d'appui présentent, vues de profil, des formes courbes avec leur convexité tournée vers l'extérieur de l'implant ;
- ladite paroi intermédiaire présente une forme courbe, avec sa convexité tournée vers l'extérieur de l'implant, et est telle qu'elle ne forme pas d'angles marqués avec lesdites parois latérales d'appui, ces parois latérales d'appui et cette paroi intermédiaire ayant ainsi, vues de profil, une forme partiellement ovale; et
- les moyens de fixation de l'implant aux vertèbres sont conçus pour permettre un assemblage non rigide de cet implant à ces vertèbres, c'est-à-dire autorisant une légère déformation de l'implant par rapport aux vertèbres lors du mouvement de ces vertèbres.

La forme courbe desdites parois latérales permet à ces parois de s'adapter précisément à la forme que présentent les faces respectives des plateaux vertébraux, assurant ainsi une certaine rétention de l'implant entre les vertèbres.

Une fois mis en place, l'implant ne fait pas obstacle aux mouvements des vertèbres compte tenu de la déformabilité de sa paroi intermédiaire ; le risque d'insertion de l'implant dans les plateaux vertébraux est dès lors fortement réduit, voire éliminé, d'autant plus que lesdites parois latérales présentent de larges surfaces de contact avec les plateaux vertébraux.

L'absence d'angles marqués entre lesdites parois latérales d'appui et ladite paroi intermédiaire permet d'éviter, sur le corps de l'implant, toute concentration des contraintes transmises par les vertèbres en un emplacement localisé de ce corps, et permet par conséquent à cet implant d'avoir une parfaite résistance à ces contraintes au cours du temps.

La forme précitée du corps de l'implant permet par ailleurs une certaine déformation de l'implant par rapport aux vertèbres lors des mouvements de ces dernières, non empêchée par lesdits moyens de fixation de cet implant aux vertèbres et seulement limitée par ceux-ci.

Cette mobilité prévient tout risque de fusion de l'espace intervertébral par suite d'une croissance des cellules osseuses autour de l'implant, et donc de conserver une totale mobilité des vertèbres l'une par rapport à l'autre au cours du temps.

De préférence, ladite paroi intermédiaire est conformée de manière, lorsqu'elle n'est pas déformée, à maintenir lesdites parois latérales d'appui à une distance l'une de l'autre légèrement supérieure à la hauteur de l'espace intervertébral à restaurer.

Cette paroi intermédiaire est donc légèrement contrainte lorsque l'implant est mis en place et permet d'assurer, par rappel élastique, un léger soutien de la vertèbre supérieure par rapport à la vertèbre inférieure.

Avantageusement, l'implant est réalisé simplement par pliage d'une même pièce de matériau approprié, notamment d'un flan de tôle métallique. Le matériau utilisé peut notamment être l'alliage de titane, d'aluminium et de vanadium connu sous la référence "TA6V".

Selon l'invention, lesdits moyens de fixation de l'implant aux vertèbres comprennent deux séries de nervures parallèles entre elles, à arêtes libres acérées, faisant saillie de la face extérieure de l'extrémité libre de chacune des parois latérales d'appui .

Ces nervures sont destinées à venir s'insérer dans la zone antérieure du corps de la vertèbre adjacente.

Pour le traitement de vertèbres cervicales, l'implant présente une paroi latérale d'appui "inférieure", c'est-à-dire venant contre la vertèbre inférieure lors de la mise en place, ayant une longueur supérieure à l'autre paroi latérale d'appui.

L'implant décrit ci-dessus peut faire partie d'une gamme d'implants comprenant au moins un autre implant intervertébral, destiné à réaliser une fusion entre les deux vertèbres à traiter ; cet autre implant, dit ci-après "de fusion", présente une structure similaire à celle de l'implant décrit ci-dessus mais comprend des moyens de fixation qui permettent son assemblage rigide autres vertèbres traitées.

De préférence, dans ce cas, lesdits moyens permettant la fixation de l'implant "de fusion" comprennent au moins une patte solidaire de l'une desdites parois latérales d'appui, percée d'un trou de réception d'une vis d'ancrage, cette vis étant destinée à être insérée dans le corps de la vertèbre correspondante.

Lesdites parois latérales d'appui de l'implant "de fusion" peuvent présenter des revêtements de surface favorisant leur ostéo-intégration et/ou comprendre des trous qui mettent l'espace qu'elles délimitent entre elles en communication avec l'extérieur de l'implant. Un greffon osseux peut alors être placé dans cet espace.

Pour sa bonne compréhension, l'invention est à nouveau décrite ci-dessous en référence au dessin schématique annexé, représentant, à titre d'exemple non limitatif, une forme de réalisation possible de l'implant intervertébral qu'elle concerne, et un implant "de fusion" inclut dans une gamme d'implants, cette gamme comprenant ledit implant intervertébral selon l'invention et ledit implant "de fusion".
La figure 1 est une vue en perspective de l'implant intervertébral qu'elle concerne ;
la figure 2 en est une vue de côté, après mise en place ;
la figure 3 est une vue en perspective dudit implant "de fusion", et
la figure 4 est une vue de côté de cet implant "de fusion", après mise en place.

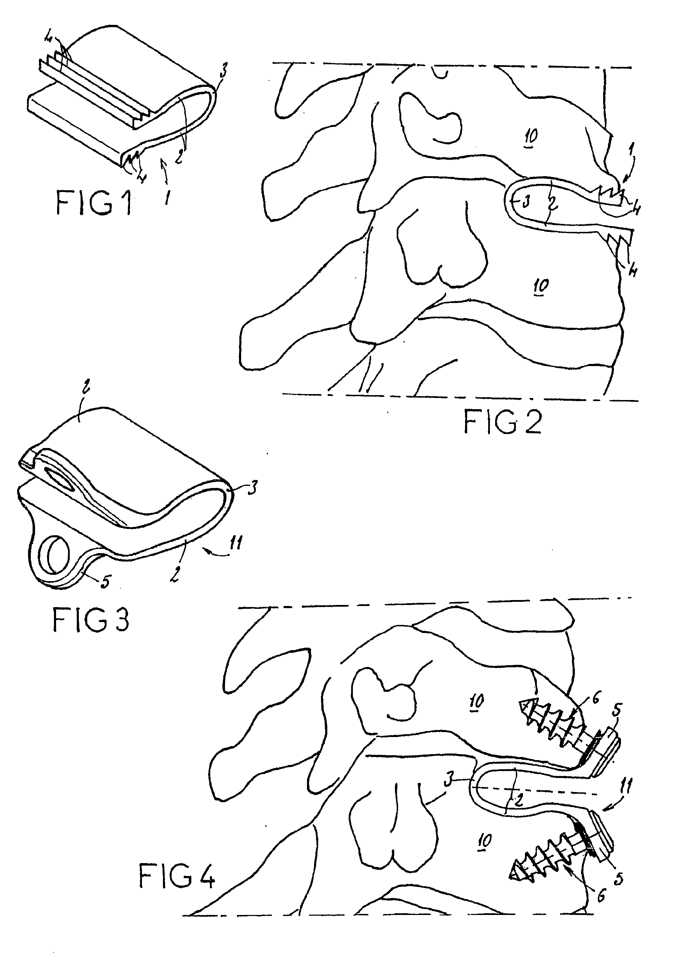

Les figures 1 et 2 représentent un implant intervertébral 1 pour le traitement de vertèbres cervicales 10 par voie d'abord antérieur.

Comme cela apparaît, l'implant 1 est réalisé par pliage d'une même pièce de matériau, et présente, vu de profil, c'est-à-dire dans le plan sagittal après implantation, une forme recourbée définissant deux parois latérales d'appui 2 et une paroi intermédiaire 3.

Ladite pièce de matériau est un flan de tôle en alliage de titane, d'aluminium et de vanadium connu sous la référence "TA6V".

Les parois latérales 2 présentent, vues de profil, des formes bombées sur la majeure partie de leur longueur, leurs convexités étant tournées vers l'extérieur de l'implant 1. Au niveau de leurs zones d'extrémité libre, ces parois latérales d'appui 2 présentent une forme rectiligne et comportent chacune une série de nervures 4.

La paroi 2 "inférieure", c'est-à-dire venant contre la vertèbre inférieure lors de l'implantation, a une longueur supérieure à l'autre paroi 2.

La paroi intermédiaire 3 présente une forme courbe dont la convexité est tournée vers l'extérieur de l'implant. Comme cela apparaît clairement, elle ne forme pas d'angles marqués avec les parois latérales d'appui 2, ces parois latérales d'appui 2 et cette paroi intermédiaire 3 ayant ainsi, vues de profil, une forme partiellement ovale, "en goutte d'eau".

La paroi intermédiaire 3 est par ailleurs déformable élastiquement entre une forme neutre, dans laquelle elle maintient normalement les parois 2 à une distance l'une de l'autre légèrement supérieure à la hauteur de l'espace intervertébral à restaurer, et une forme contrainte, dans laquelle cette paroi 3 autorise le rapprochement des extrémités libres des deux parois 2. Ce rapprochement est tel qu'il permet de diminuer la hauteur de l'implant 1 de telle sorte que cette hauteur soit inférieure à la hauteur de l'espace intervertébral à restaurer.

Les nervures 4 sont parallèles entre elles et font saillie de la zone d'extrémité libre de chaque paroi 2, vers l'extérieur de l'implant 1. Chacune d'elles est délimitée par une face antérieure perpendiculaire à la direction longitudinale de l'implant 1 et par une face postérieure inclinée, formant un angle d'environ de 50° avec la face antérieure. Ces nervures 4 présentent ainsi des arêtes libres relativement acérées.

L'implant 1 représenté à titre d'exemple présente les dimensions suivantes :
- dimension maximale de l'implant dans le plan sagittal : environ 17mm;
- différence de longueur des parois 2 : environ 1 mm ;
- dimension de l'implant dans le plan frontal : environ 18 mm ;
- épaisseur dudit flan au niveau des parois 2 et de la paroi 3 : environ 1 mm ;
- épaisseur maximum de l'implant 1, au niveau des faces extérieures bombées des parois latérales 2 : environ 7 mm ;
- rayon de courbure de la moitié supérieure de la paroi intermédiaire 3 : environ 2,7 mm ;
- rayon de courbure de la moitié inférieure de la paroi intermédiaire 3 : environ 3,3 mm ;
- rayon de courbure de la zone bombée de la paroi latérale 2 supérieure: 10 mm ;
- rayon de courbure de la zone bombée de la paroi latérale 2 inférieure : environ 25 mm ;

En pratique, les parois 2 sont rapprochées l'une de l'autre par déformation de la paroi 3, pour permettre l'insertion de l'implant 1 entre les plateaux vertébraux des deux vertèbres 10 à traiter, puis, une fois cette insertion réalisée, les parois 2 sont relâchées, ce qui les plaque contre ces plateaux vertébraux. Les nervures 4 sont insérées dans les plateaux vertébraux et permettent un assemblage non rigide de l'implant 1 à ces vertèbres ou tissus, c'est-à-dire autorisant une légère déformation de l'implant par rapport aux vertèbres lors du mouvement de ces vertèbres, tout en s'opposant à toute expulsion de l'implant.

La forme bombée des parois 2 permet à ces parois de s'adapter précisément à la forme que présentent les faces respectives de ces plateaux vertébraux, et assure une certaine rétention de l'implant entre les vertèbres 10. La contrainte élastique subsistant dans la paroi 3 permet de maintenir les nervures 4 insérées dans les vertèbres 10.

L'implant "de fusion" 11 montré sur les figures 3 et 4 présente une structure similaire à celle de l'implant 1 décrit ci-dessus, sinon que les parois 2 comprennent deux pattes 5, solidaires d'elles et prolongeant leurs extrémités libres.

Chacune de ces pattes 5 est rattachée à l'extrémité de la paroi 2 qui la comporte par deux zones de raccordement latérales courbes, qui permettent d'assurer une liaison parfaitement solide de cette patte 5 et de la paroi 2, et est percée d'un trou de réception d'une vis d'ancrage 6. Cette vis 6 est destinée à être insérée dans le corps de la vertèbre 10 correspondante, comme le montre la figure 4.

Chaque patte 5 forme un angle de l'ordre de 120° avec la direction antéro-postérieure générale de la paroi 2 à laquelle cette patte est rattachée, et présente une épaisseur supérieure à celle du reste de l'implant 1. Cette épaisseur est d'environ 1,5 mm dans l'exemple représenté.

L'implant "de fusion" 11 est utilisé pour réaliser une fusion entre les deux vertèbres 10 à traiter.

Il apparaît de ce qui précède que l'invention apporte une amélioration déterminante à la technique antérieure, en fournissant un implant intervertébral permettant de parfaitement restaurer l'espace intervertébral, sans former un obstacle aux mouvements des vertèbres, sans induire de risques d'insertion dans les plateaux vertébraux ni de risque de fusion par croissance des cellules osseuses, en étant facile à implanter et en ayant une pérennité non sujette à caution.

Il va de soi que l'invention n'est pas limitée à la forme de réalisation décrite ci dessus à titre d'exemple mais qu'elle en embrasse au contraire toutes les variantes de réalisation couvertes par les revendications ci-annexées.

## Revendications

1. Implant intervertébral (1), notamment destiné au traitement de vertèbres cervicales (10) par voie d'abord antérieur, comprenant deux parois latérales (2) d'appui contre les plateaux vertébraux et une paroi intermédiaire (3) de raccordement de ces parois latérales d'appui, cet implant (1) étant déformable élastiquement pour son insertion entre les vertèbres (10) à traiter et pour permettre de restaurer une mobilité amortie de ces vertèbres (10), et comprenant des moyens (4) pour son assemblage à ces vertèbres (10);
- lesdites parois latérales d'appui (2) présentant, vues de profil, des formes courbes avec leur convexité tournée vers l'extérieur de l'implant (1);
- ladite paroi intermédiaire (3) présentant une forme courbe, avec sa convexité tournée vers l'extérieur de l'implant (1), et est telle qu'elle ne forme pas d'angles marqués avec lesdites parois latérales d'appui (2), ces parois latérales d'appui (2) et cette paroi intermédiaire (3) ayant ainsi, vues de profil, une forme partiellement ovale;
- les moyens (4) de fixation de l'implant (1) aux vertèbres (10) comprenant deux séries de nervures parallèles entre elles, à arêtes acérées, et faisant saillie de la face extérieure de l'extrémité libre de chacune des parois latérales d'appui ; implant (1) **caractérisé en ce que**:
- ladite paroi latérale (2) inférieure ayant une longueur supérieure à l'autre paroi latérale (2).

2. Implant (1) selon la revendication 1, **caractérisé en ce que** ladite paroi intermédiaire (3) est conformée de manière, lorsqu'elle n'est pas déformée, à maintenir lesdites parois latérales d'appui (2) à une distance l'une de l'autre légèrement supérieure à la hauteur de l'espace intervertébral à restaurer.

3. Implant (1) selon la revendication 1 ou la revendication 2, **caractérisé en ce qu'**il est réalisé par pliage d'une même pièce de matériau approprié, notamment d'un flan de tôle métallique.

4. Implant (1) selon la revendication 3, **caractérisé en ce que** le matériau utilisé est l'alliage de titane, d'aluminium et de vanadium connu sous la référence "TA6V".

## Patentansprüche

1. Zwischenwirbelimplantat (1), insbesondere zum Behandeln von Halswirbeln (10) über den anterioren Zugang, umfassend zwei seitliche Stützwände (2) an den Wirbelplatten und eine Zwischenwand (3) zum Verbinden dieser seitlichen Stützwände, wobei das Implantat (1) zum Einfügen desselben zwischen die zu behandelnden Wirbel (10), und um eine abgeschwächte Beweglichkeit dieser Wirbel (10) wiederherstellen zu können, elastisch verformbar ist, und umfassend Mittel (4) zum Zusammenfügen desselben an diesen Wirbeln (10);
- wobei die seitlichen Stützwände (2) im Profil gesehen gekrümmte Formen aufweisen, wobei ihre Konvexität zur Außenseite des Implantats (1) gewandt ist;
- wobei die Zwischenwand (3) eine gekrümmte Form aufweist, wobei ihre Konvexität zur Außenseite des Implantats (1) gewandt ist, und derart gestaltet ist, dass sie keine ausgeprägten Winkel mit den seitlichen Stützwänden (2) bildet, wobei diese seitlichen Stützwände (2) und diese Zwischenwand (3) somit im Profil gesehen eine teilweise ovale Form aufweisen;
- wobei die Mittel (4) zum Befestigen des Implantats (1) an den Wirbeln (10) zwei Reihen von zueinander parallelen Rippen mit scharfen Kanten umfassen, welche von der Außenseite des freien Endes jeder der seitlichen Stützwände vorstehen;
wobei das Implantat (1) **dadurch gekennzeichnet ist, dass**:
- die untere seitliche Wand (2) eine Länge aufweist, die größer als die der anderen seitlichen Wand (2) ist.

2. Implantat (1) nach Anspruch 1, **dadurch gekennzeichnet, dass** die Zwischenwand (3) derart gestaltet ist, dass sie, wenn sie nicht verformt ist, die seitlichen Stützwände (2) in einem Abstand voneinander hält, der etwas größer als die Höhe des wiederherzustellenden Zwischenwirbelraums ist.

3. Implantat (1) nach Anspruch 1 oder 2, **dadurch gekennzeichnet, dass** es durch Biegen ein und desselben Teils aus einem geeigneten Material, insbesondere einem Metallblechzuschnitt, ausgebildet ist.

4. Implantat (1) nach Anspruch 3, **dadurch gekennzeichnet, dass** das verwendete Material die Legierung aus Titan, Aluminium und Vanadium ist, die unter der Bezeichnung "TA6V" bekannt ist.

## Claims

1. An intervertebral implant (1), in particular for the treatment of cervical vertebrae (10) by anterior approach, comprising two lateral supporting walls (2) bearing against the vertebral plates and an intermediate wall (3) joining said lateral supporting walls, said implant (1) being elastically deformable for its insertion between the vertebrae (10) to be treated and for restoring an attenuated mobility of said vertebrae (10), and comprising means (4) for the mounting thereof to said vertebrae (10);
- said lateral supporting walls (2), when seen from the side, having a curved shape, the convexity thereof being oriented towards the outside of the implant (1);
- said intermediate wall (3) having a curved shape, the convexity thereof being oriented towards the outside of the implant (1) and is such that it does not form any pronounced angles with said lateral supporting walls (2), said lateral supporting walls (2) and said intermediate wall (3), when seen from the side, thus having a partially oval shape;
- said means (4) for fixing the implant (1) to the vertebrae (10) comprising two series of ribs parallel to one another, with sharp edges, and protruding from the external side of the free end of each of the lateral supporting walls;
wherein the implant (1) is **characterized in that**:
- said lower lateral wall (2) having a larger length than the other lateral wall (2).

2. The implant (1) according to claim 1, **characterized in that** said intermediate wall (3) is shaped so as to keep said lateral supporting walls (2) at a distance from one another slightly larger than the height of the intervertebral space which is to be restored, when it is not deformed.

3. The implant (1) according to claim 1 or 2, **characterized in that** it is formed by bending of a same piece of suitable material, in particular of a sheet metal blank.

4. The implant (1) according to claim 3, **characterized in that** the material which is used is the alloy of titanium, aluminium, and vanadium, known under the reference "TA6V".
